# EUROPEAN PATENT APPLICATION

(11) **EP 0 928 598 A2**
(43) Date of publication of application: **14.07.1999**
(21) Application number: 98124408.0
(22) Date of filing: 21.12.1998
(51) Int. Cl.: A61B 5/0215, A61F 2/06, A61B 17/12

(54) **Device for fixation of a sensor in a bodily lumen**

(30) Priority: 08.01.1998 US 4420
(71) Applicant: Microsense Cardiovascular, Systems (1996) Ltd., Givat Ela 23800 (IL)
(72) Inventor: Richter, Jacob, Ramat Hasharon 47226 (IL); Kaplan, Shay, Givat Ela 23800 (IL)
(74) Representative: Altenburg, Udo, Dipl.-Phys.

(57) **Abstract**

A device and method for fixation of a sensor in a bodily lumen, in which a sensor support is coupled to a fixation device, which is inserted into a bodily lumen. The fixation device is then secured within the bodily lumen, for example, by expansion or by suturing to the bodily lumen. The fixation device may be a stent or a dedicated anchoring ring having a sensor support coupled thereto. The fixation device may be inserted during an intervention procedure or during a special insertion procedure. The sensor may be remotely interrogated exterior to the bodily lumen periodically or continuously. Furthermore, the sensor may be provided with a protective coating to protect it from damage during insertion.

## Description

### FIELD OF THE INVENTION

The present invention relates generally to a method and device for fixation of a sensor in a bodily lumen and for protection of the sensor during insertion into a bodily lumen.

### BACKGROUND OF THE INVENTION

Sensors for the monitoring and/or recordation of various human physical, chemical and/or physiological parameters are known in the art. U.S. Patent No. 4,485,813 describes a sensor that may be permanently implanted in a specific location within the human body in an implantable medical device such as a pacemaker. This sensor is used to monitor certain physical and/or physiological parameters of the subject in which it has been implanted. This sensor can be maintained in the subject for extended periods of time to continuously monitor information about the subject.

A severe limitation to the sensor described in U.S. Patent No. 4,485,813 is the limited number of possible locations in which it can be implanted due to the requirement that the sensor be located in a medical device such as a pacemaker and the difficulty of fixation independently. This limitation on the location and fixation of the sensor limits the usefulness of the sensor for inter-lumen applications.

Sensors used to monitor parameters within lumens are made of very thin membranes that are highly sensitive to mechanical pressure. As a result there is a great risk of the sensor being damaged during insertion and or positioning. Damage to the sensor could result in poor performance or non-operability of the sensor. For example, should the membrane of a sensor break during insertion, the sensor would be rendered inoperable. Due to the risks associated with the procedures for the insertion of sensors, there would be great costs and risks involved should a sensor be damaged or destroyed during insertion. Thus, there is also a need for a device and method of protecting sensors during insertion and fixation.

### SUMMARY OF THE PRESENT INVENTION

It is, therefore, an object of the present invention to provide a method and device for fixation of a sensor in a bodily lumen. Through the use of such a method and device, remotely interrogated sensors may be fixed within bodily lumens. Such sensors may be used to record and/or monitor parameters such as, for example, physiological parameters, *e.g*., pressure and velocity of flow, and biochemical parameters, *e.g*., level of gases and biochemical substances in the fluid contained in the lumen.

The monitoring of conditions in lumens today dictates some level of intervention and the fiequency of such monitoring is limited by the relative risk of the required intervention. The present invention, therefore, provides a sensor device which may be implanted, either temporarily or permanently, in a lumen and interrogated from an exterior position, for example, the surface of the body, at any time without any intervention or physical intrusion.

The present invention provides a method and device for the fixation of such sensors in specific desired locations and/or preferred positions in the lumen. Such fixation of the sensors may be achieved at the time of any required surgical intervention or independently by catheterization. Furthermore, the sensor may be connected to the repair device, *e.g*., the stitches of a bypass, an aneurismal repair device, a stent, etc., or mounted on its own dedicated fixation device.

A sensor may be fixed inside a lumen by any number of means, including directly attaching the sensor in place, for example, by including holes in the sensor, *e.g.*, around its periphery, and attaching the sensor to the stitches of a bypass during surgery, or through the use of a surgical adhesive. A sensor may also be positioned inside a lumen using a carrier or support (of any shape and size) which may be part of, or coupled to a repair device, *e.g*., a stent or aneurismal correction device which holds the sensor in place adjacent to or near the repair device. Additionally, a sensor may be positioned inside a lumen using a dedicated device, *e.g*., an anchoring ring, which is held within a lumen and fixed in place, for example, by expansion with a catheter balloon. The anchoring ring does not necessarily have to be circular in shape, but may instead be oval or any other shape best suited for the location where placed. Additionally, the anchoring ring may have a separate carrier or support to hold the sensor. The carrier or support may be any shape or size, including, for example, circular, square, rectangular, diamond shaped, linear with or without a bent or curved end, etc, and it may be constructed as only a border or as a solid piece of material.

Multiple sensors may be attached to a carrier or carriers, for example, two sensors with one placed on each side of a stent, or two sensors attached at both connections of a bypass section, *e.g*., one sensor at the entrance to an aneurismal sleeve and one at the outside of the sleeve to monitor for a possible leak around the sleeve. Additionally, a sensor may have multiple repair devices or dedicated devices supporting it within a lumen, either with or without a carrier, for example, a sensor supported between two anchoring rings

A sensor may be supported by or connected to a carrier, for example, by providing a groove-like depression(s) or notch-like depression(s) in the sensor into which a portion(s) of the carrier may be inserted, or the sensor may be configured such that a portion(s) of the sensor, for example, a lip-like extension(s) or protrusion(s), may extend beyond the dimensions of the carrier to be supported thereby. Additionally, the sensor may be attached to the carrier, for example, by welding and/or glueing or any combinations of the above.

After a sensor is fixed within a lumen, for example, during an intervention procedure such as aneurismal device implantation, PTCA, coronary bypass surgery, etc., it may thereafter be monitored periodically to track any of a variety of parameters or to assess the effectiveness of the procedure that was performed. For example, the sensor may be monitored periodically to assess the long term progress or deterioration of the corrective effect, and the progress of relevant symptoms of a disease.

Multiple sensors may be implanted and may be monitored individually or simultaneously to derive gradients along a lumen and across a repair device or section. Such sensors may be fixed in any number of positions within a lumen, for example, on both sides of a lesion treated by PTCA with or without a stent, on both sides of a bypass section, and before, after and around an aneurismal repair device, etc.

The present invention also provides for a device and method for the protection of sensors during insertion. In order to preserve sensors during insertion and remove the risk of damage to or destruction of sensors during an insertion or positioning procedure, sensors may be coated with a protective layer which is soluble in an aqueous solution, and which disappears immediately or soon alter deployment of the sensor in the body. The material used for, thickness of, and hardness of the coating may vary, for example, depending on the location of the sensor, the type of sensor, protection level sought, and rate of dissolution desired.

The fixation device may be constructed by first creating a flat version of the desired pattern for the fixation device, for example, from a piece of thin stainless steel sheet metal or some other material, *e.g.*, any metal, non-metallic or bioabsorbable material. The flat pattern can be produced through any suitable technique, such as etching the design into the sheet metal, by cutting with a very fine laser, or by any other technique.

Once the material has been cut, it is deformed so as to cause its edges to meet. To create a fixation device from a flat, metal pattern, the flat metal is rolled until the edges meet. The portion which holds the sensor may be located along the circumference of the fixation device, may extend perpendicular to the cross-section of the ring formed or may extend in some other manner from the ring formed by the fixation device. The locations where edges meet are joined together, such as by spot welding. Afterwards, the fixation device is polished, either mechanically or electrochemically.

### BRIEF DESCRIPTION OF THE DRAWINGS

The present invention will be understood and appreciated more fully from the following detailed description taken in conjunction with the drawings in which:
Fig. 1A is a drawing of a first fixation device for a sensor according to a preferred embodiment of the present invention;
Fig. 1B is an illustration of a method for fixation of the sensor of Fig. 1A within a lumen according to a preferred embodiment of the present invention;
Figs. 2A and 2B, show illustrations of a second fixation device for a sensor before expansion and after expansion, respectively, according to a preferred embodiment of the present invention.
Figs. 3A and 3B, show illustrations of a third fixation device for a sensor before expansion and after expansion, respectively, according to a preferred embodiment of the present invention.
Fig. 4, illustrates a second method for fixation of a sensor within a lumen using the third fixation device shown in Figs. 3A and 3B, according to a preferred embodiment of the present invention.
Fig. 5, shows an illustration of a mask for etching of a flat design of the fixation device of Figs. 3A and 3B, according to a preferred embodiment of the present invention.
Fig. 6, shows an illustration of a mask for etching of a flat design of the fixation device of Figs. 2A and 2B, according to a preferred embodiment of the present invention.
Figs. 7, shows a fourth fixation device for a sensor before expansion, according to a preferred embodiment of the present invention.
Fig. 8, shows an illustration of a mask for etching of a flat design of the fixation device of Fig. 7, according to a preferred embodiment of the present invention.
Figs. 9A and 9B, show an enlarged side view of a cross section of the sensor support from Fig. 3A along the line formed between points a' and a' according to two different embodiments of the present invention.

### DETAILED DESCRIPTION OF A PREFERRED EMBODIMENT

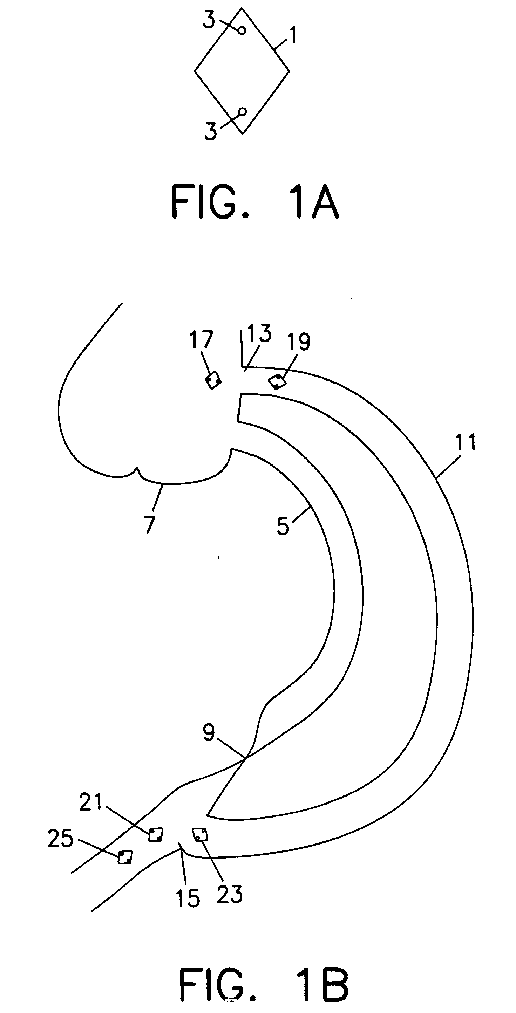

Reference is now made to Figs. 1A and 1B, which illustrate a first fixation device for a sensor and a first method for fixation of a sensor within a lumen, respectively, according to a preferred embodiment of the present invention.

In Fig. 1A, there is shown a sensor 1 having two holes 3 in its periphery for attachment to sutures within a lumen. In Fig. 1B, there is shown a coronary artery 5 starting at the Aorta 7 and having an occlusion 9. A bypass 11 is connected between the Aorta at point 13 and at point 15 beyond the occlusion 9. Sensor 1 is placed either at the proximal ostium 17 or at the proximal part of the bypass 19. Alternatively, sensor 1 may be placed at the distal ostium 21, at the distal part before the distal ostium 23, or at the distal part after the distal ostium 25. Any number of sensors may be used, and they may be placed in any combination of the above positions or any other position desired. The sensor 1 is fixed in place using the two holes 3 for attachment to the sutures. Alternatively, the sensor 1 may be fixed in place using surgical adhesive or a surgical staple(s).

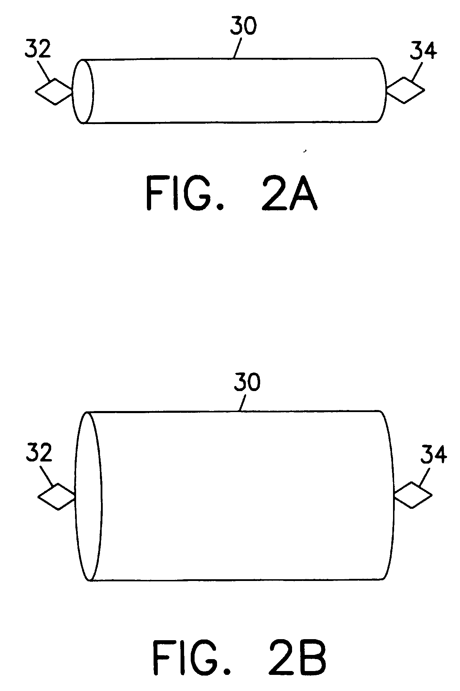

Referring now to Figs. 2A and 2B, there are shown a second fixation device for a sensor before expansion and after expansion, respectively, according to a preferred embodiment of the present invention.

In Fig. 2A, there is shown a stent 30 in a non-expanded state with a first sensor support 32 and a second sensor support 34. Alternatively, the stent 30 may include only one or more than two sensor supports. For example, a third sensor support may be located opposite the first sensor support 32. In Fig. 2B, the stent 30 from Fig. 2A is shown in its expanded state. Expansion may be accomplished, for example, by balloon catheterization or some other procedure. To fix a sensor within a lumen, the stent 30 is positioned as it normally would be during any medical procedure in which a stent is used. Prior to expansion, and either prior to or after insertion of the stent 30 into the lumen, a sensor is placed in, placed on or attached to the first sensor support 32 and/or the second sensor support 34. The stent 30 is then either expanded, or inserted into the lumen and then expanded. The same procedure may be used to fix any number of sensors within a lumen, with the additional step of placing each sensor either in or on, or attaching each sensor to its corresponding sensor support.

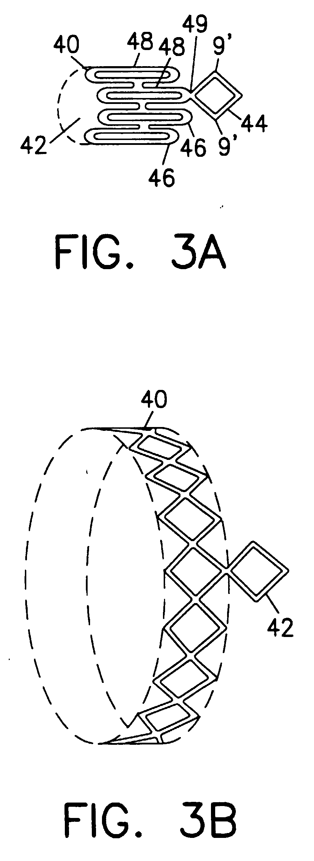

Referring now to Figs. 3A and 3B, there are shown a third fixation device for a sensor before expansion and after expansion, respectively, according to a preferred embodiment of the present invention.

In Fig. 3A, there is shown a fixation device 40 in the form of an anchoring ring 42, in a non-expanded state coupled to a sensor support 44. The fixation device 40 may be formed of any malleable material which does not revert automatically to its original shape after being expanded. The anchoring ring 42 is made up of a plurality of elliptical sections 46 connected one to the other at the middle of each of their long portions 48 to form a ring. The sensor support 44 is connected to one of the elliptical sections 46 at a short portion 49, and perpendicular to a cross-section of the anchoring ring 42 forming a circular plane. The sensor support 44 is formed in the shape of a diamond, but can be any shape desired. Additionally, there may be multiple sensor supports attached to the anchoring ring 42. Alternatively, the anchoring ring 42 may be made of a single sinusoidal ring, with one or more sensor supports attached to the peaks, since it does not serve any support function for the lumen.

Fig. 3B shows the fixation device 40 of Fig. 3A in an expanded state. To fix a sensor within a lumen, the fixation device 40 is positioned within the lumen, for example, during an intervention procedure, and expanded, for example, by balloon catheterization or some other procedure. Prior to expansion, and either prior to or after insertion of the fixation device 40 into the lumen, the sensor is placed in, placed on or attached to the sensor support 42. The fixation device 40 is then either expanded, or inserted into the lumen and then expanded. The same procedure may be used to fix multiple sensors within a lumen, with the additional step of placing each sensor either in or on, or attaching each sensor to a corresponding sensor support.

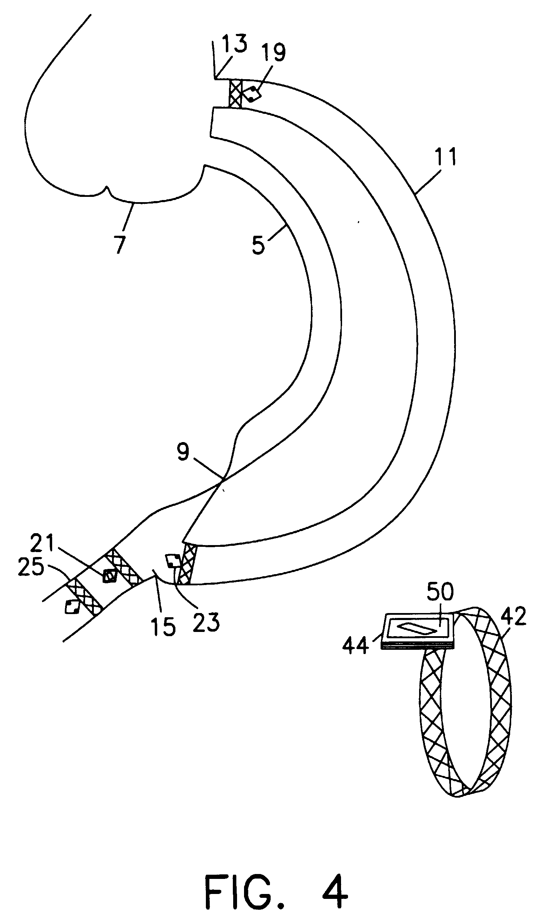

Referring now to Fig. 4, which illustrates a second method for fixation of a sensor within a lumen using the third fixation device shown in Figs. 3A and 3B, according to a preferred embodiment of the present invention. As illustrated in Fig. 1B, a coronary artery 5 starting at the Aorta 7 and having an occlusion 9 is fitted with a bypass 11 which is connected between the Aorta at point 13 and at point 15 beyond the occlusion 9. Sensor 50, which is carried by the sensor support 44 coupled to the anchoring ring 42 of Figs. 3A and 3B, is placed either at the proximal part of the bypass 19, at the distal ostium 21, at the distal part before the distal ostium 23, or at the distal part after the distal ostium 25. Any number of sensors may be used, and they may be placed in any combination of the above positions or any other position desired in which an anchoring ring can be used. The sensor 50 is fixed in place by expansion using balloon catheterization.

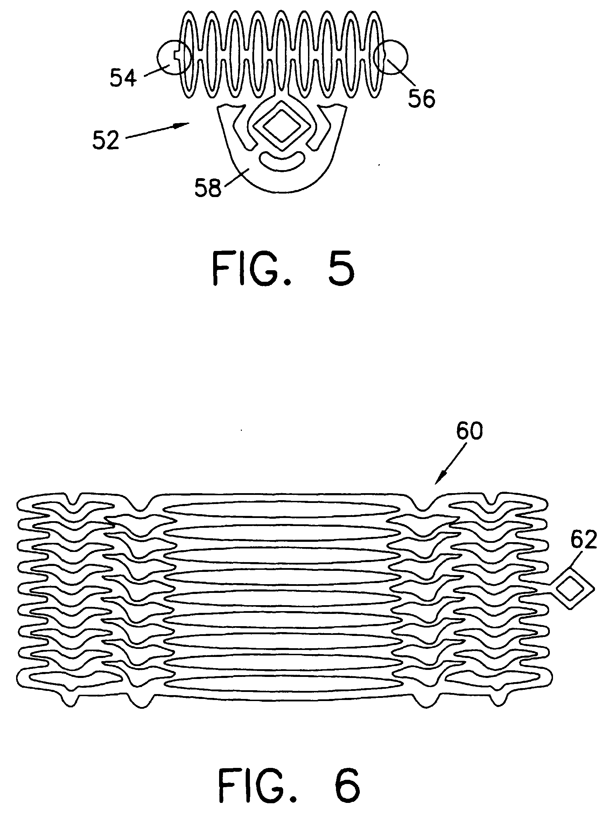

Referring now to Fig. 5, there is shown an illustration of a mask for etching of a flat design of the fixation device of Figs. 3A and 3B, according to a preferred embodiment of the present invention. A mask 52 is created for etching a flat design of a fixation device. The flat design is then etched onto a piece of thin sheet metal or some other malleable material. The flat design is next cut from the sheet metal using, for example, a fine laser. The cut flat design is then polished and bent into a circular (or other) shape. Points 54 and 56 show the locations where the flat design is coupled, for example, by welding after it is bent. The welding creates an anchoring ring. Sensor support 58 is positioned approximately at the midpoint of the mask 52, but may alternatively be located at any other position. Additionally, there may be multiple sensor supports, for example, located at both sides of the fixation device design.

Referring now to Fig. 6, there is shown an illustration of a mask for etching of a flat design of the fixation device of Figs. 2A and 2B, according to a preferred embodiment of the present invention. A mask 60 is created for etching a flat design of a stent. The flat design is then etched onto a piece of thin sheet metal or some other malleable material. The flat design is next cut from the sheet metal using, for example, a fine laser. The cut flat design is then polished and bent into a circular (or other) shape and coupled, for example, by welding after it is bent. Sensor support 62 is positioned approximately at the midpoint of the mask 60, but may alternatively be located at any other position. Additionally, there may be multiple sensor supports, for example, located at both sides of the stent design.

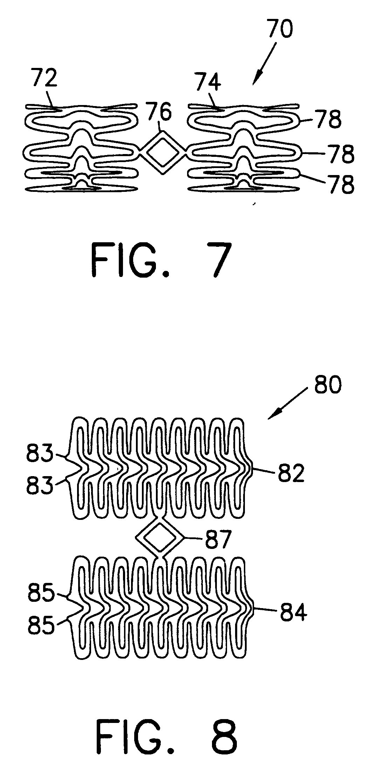

Referring now to Fig. 7, there is shown a fourth fixation device for a sensor before expansion, according to a preferred embodiment of the present invention. A fixation device 70 in the form of a dual anchoring ring comprises a first ring 72 and a second ring 74, in a non-expanded state, with a sensor support 76 positioned between the two rings 72, 74. The fixation device 70 may be formed of any malleable material which does not revert automatically to its original shape after being expanded. The fixation device 70 is made up of a plurality of sections 78 connected one to the other to form two anchoring rings 72, 74. A sensor support 76 is connected to one of the sections 78 of each anchoring ring 72, 74 perpendicular to a cross-section of each of the rings 72, 74 forming a circular plane, and is positioned between the two rings 72, 74. The sensor support is formed in the shape of a diamond, but can be any shape desired. Additionally, there may be multiple sensor supports attached to the fixation device 70. Alternatively, the fixation device 70 may be made of two single sinusoidal rings, with one or more sensor supports attached to the peaks, since it does not serve any support function for the lumen. The fixation device 70 may alternatively be made of two stents, one on each side of a sensor support, or having multiple sensor supports attached thereto.

Referring now to Fig. 8, there is shown an illustration of a mask for etching of a flat design of the fixation device of Fig. 7, according to a preferred embodiment of the present invention. A mask 80 is created for etching a flat design of a fixation device. The flat design is then etched onto a piece of thin sheet metal or some other malleable material. The flat design is next cut from the sheet metal using, for example, a fine laser. The cut flat design is then polished and bent into a circular (or other) shape. Points 82 and 83, and points 84 and 85 show the respective locations where the flat design is coupled, for example, by welding after it is bent. The welding creates two anchoring rings. Sensor support 87 is positioned approximately at the midpoint of the mask 80, but may alternatively be located at any other position. Additionally, there may be multiple sensor supports, for example, located at both sides of the fixation device design.

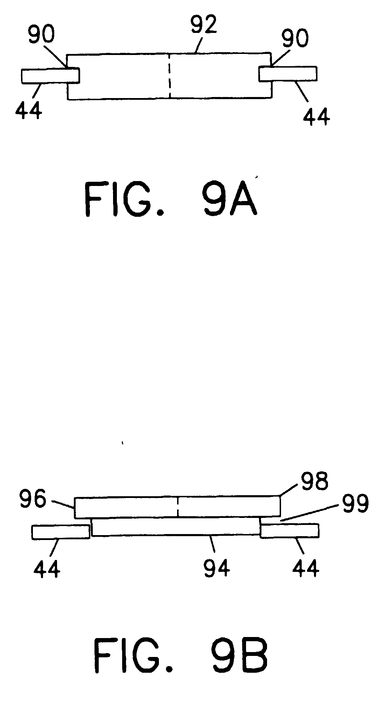

Referring now to Figs. 9A and 9B, there is shown an enlarged side view of a cross section of the sensor support from Fig. 3A along the line formed between points a' and a'. As shown in Fig. 9A, a groove 90 is formed in two portions of the periphery of sensor 92, for example, by cutting with a wire saw, by etching, by laser cutting, etc., and the sensor 92 is then inserted into the sensor support 44 such that two portions of the sensor support 44 are positioned within the groove 90 providing support for the sensor 92. Alternatively, instead of the grooves, two notches may be formed in the periphery of the sensor 92 in which the two portions of the sensor support 44 may be positioned.

As shown in Fig. 9B, sensor 94 is formed with a lip 96 around its upper edge 98. Sensor 94 may instead be formed with one or more protrusions along its upper edge 98. Alternatively, the lip or protrusion(s) may be located on the bottom or at any other position on the sensor. The sensor 94 is coupled to the sensor support 44, for example, by glueing, welding, soldering, etc., the lip 96 or protrusion(s) to an edge or portion 99 of the sensor support. Alternatively, the sensor 94 may be placed on the sensor support 44 and supported by the lip 96 or by the protrusion(s).

Due to the sensitivity of the sensors that are used for monitoring, which have very thin membranes that are extremely sensitive to mechanical pressure, a coating may be placed on the sensors to protect them from damage and/or destruction during deployment. The coating may be made from a material that is soluble in an aqueous solution, and should dissolve immediately or soon after deployment of the sensor. The material used, the thickness of the coating and the hardness of the coating will depend to a large extent on the location of the sensor, the type of sensor, and a variety of other factors including the physiology involved, the parameters being measured, and the desired speed of deployment.

A first example of a coating is a composition comprising solidified sugar syrup made of approximately equal amounts of glucose and sucrose. The proportions of glucose and sucrose may be varied, however, depending on the application.

A second example of a coating is a composition comprising Hydroxy Propyl Methyl Cellulose, Hydroxy Propyl Cellulose and Colloidal Silicone Dioxide, all finely ground and mixed in water, which is used for coating pills and is commercially available as Opadry-Oy-34817 from Colorcon Ltd., Italy.

Other materials may be used as a protective coating for a sensor. The protective coating may be made from any other substance which is hard or thick enough to protect the sensor from damage during insertion, dissolves immediately or soon after insertion and is biocompatible in the intended location of deployment in the body.

A sensor may be coated by any available method for coating objects including, for example, spraying the coating on the sensor, dipping the sensor in a liquid bath, pouring or dripping the coating onto the sensor, painting the coating onto the sensor, etc. Additionally, the coating may cover only the membrane of the sensor or it may cover a larger portion of the sensor or the entire sensor.

## Claims

1. Apparatus for fixation of a sensor in a bodily lumen, comprising:
a fixation device; and
a sensor support coupled to the fixation device.

2. The apparatus of claim 1, wherein the fixation device is a stent.

3. The apparatus of claim 2, wherein the stent having an end, and the sensor support coupled to the end of the stent.

4. The apparatus of claim 1, wherein the fixation device is an aneurismal repair device.

5. The apparatus of claim 1, wherein the fixation device is an anchoring ring.

6. The apparatus of claim 5, wherein the anchoring ring further comprising at least one piece of material forming a parameter and arranged as at least one sinusoid positioned perpendicular to a plane formed by a cross section of the anchoring ring.

7. The apparatus of claim 6, wherein the sensor support is coupled to a peak of the sinusoid of the anchoring ring.

8. The apparatus of claim 5, wherein the anchoring ring further comprising a plurality of ellipses, each having long portions and short portions, joined one to the other at approximately mid-points of the long portions.

9. The apparatus of claim 8, wherein the sensor support is coupled to a ellipse of the anchoring ring at approximately a mid-point of one of the short portions.

10. The apparatus of claim 1, wherein the fixation device includes at least a first anchoring ring and a second anchoring ring, and the sensor support coupled between the first anchoring ring and the second anchoring ring.

11. The apparatus of claim 1, wherein the fixation device includes at least a first stent and a second stent, and the sensor support coupled between the first stent and the second stent.

12. The apparatus of claim 1, wherein the sensor support comprises at least a first sensor support and a second sensor support displaced apart from one another within the lumen.

13. The apparatus of claim 12, wherein the fixation device having a first end and a second end, the first sensor support coupled generally adjacent to the first end and the second sensor support coupled generally adjacent to the second end of the fixation device.

14. The apparatus of claim 1, wherein the fixation device is expandable.

15. The apparatus of claim 1, wherein the sensor has at least one notch-like depression disposed in its periphery and wherein at least one portion of the sensor support is positioned within the at least one notch-like depression.

16. The apparatus of claim 1, wherein the sensor has a groove-like depression at at least one portion of its periphery and wherein at least one portion of the sensor support is positioned within the groove-like depression.

17. The apparatus of claim 1, wherein the sensor having a lip-like extension at at least one portion of its periphery and wherein at least one portion of the lip-like extension extending beyond the inner-most portion of the sensor support.

18. The apparatus of claim 1, wherein the sensor having at least one protrusion disposed on its periphery and wherein at least one portion of the at least one protrusion extending beyond the inner-most portion of the sensor support..

19. Apparatus for fixation of a sensor in a bodily lumen, comprising:
a fixation device forming a perimeter having a discernable width, the fixation device having a first end and a second end; and
at least one sensor carrier coupled to the first end of the fixation device and extending generally parallel to the perimeter in a direction away from the second end,
wherein the sensor is supported by the sensor carrier.

20. Apparatus for fixation of a sensor in a bodily lumen, comprising a sensor having at least two holes extending therethrough.

21. Apparatus for fixation of a sensor in a bodily lumen, comprising a sensor having a border, the border including at least two holes extending therethrough.

22. The apparatus of claim 21, wherein each hole is disposed on a different side of the border.

23. The apparatus of claim 21, wherein each hole is approximately equidistant from the other.

24. A method for fixation of a sensor in a bodily lumen, comprising the steps of:
placing the sensor onto a sensor support coupled to a fixation device;
inserting the fixation device into a bodily lumen; and
securing the fixation device within the bodily lumen.

25. A method for fixation of a sensor in a bodily lumen, comprising the steps of:
placing the sensor into a sensor support coupled to a fixation device;
inserting the fixation device into a bodily lumen; and
securing the fixation device within the bodily lumen.

26. The method according to claim 24, wherein the fixation device is a stent.

27. The method according to claim 24, wherein the fixation device is an aneurismal repair device.

28. The method according to claim 24, wherein the fixation device is an anchoring ring.

29. The method according to claim 24, further comprising the step of coupling the sensor to the sensor support.

30. The method according to claim 29, wherein the coupling includes gluing the sensor to the sensor support.

31. The method according to claim 29, wherein the coupling includes welding the sensor to the sensor support.

32. The method according to claim 25, further comprising the step of coupling the sensor to the sensor support.

33. The method according to claim 32, wherein the coupling includes positioning at least one portion of the sensor support in at least one groove-like depression in the sensor.

34. The method according to claim 32, wherein the coupling includes positioning at least one portion of the sensor support in at least one notch-like depression in the sensor.

35. The method according to claim 24, wherein the inserting is accomplished during an intervention procedure.

36. The method according to claim 24, wherein the securing is accomplished by expanding the fixation device.

37. The method according to claim 36, wherein the expanding is accomplished by balloon catheterization.

38. A method for fixation of a sensor in a bodily lumen, comprising the steps of:
inserting the sensor into a bodily lumen; and
coupling the sensor to a section of the bodily lumen.

39. The method of claim 38, wherein the sensor is coupled to the section of the bodily lumen using sutures.

40. The method of claim 38, wherein the sensor is coupled to the section of the bodily lumen using adhesive.

41. A method for constructing an apparatus for fixation of a sensor in a bodily lumen, comprising the steps of:
forming a mask;
etching a flat design of both a fixation device and a sensor support on a portion of material, the flat design of the fixation device having a first end and a second end; and
coupling the first end and the second end of the flat design of the fixation device.

42. The method according to claim 41, wherein the fixation device is a stent.

43. The method according to claim 41, wherein the material is thin sheet-metal.

44. The method according to claim 41, wherein the material is stainless steel.

45. The method according to claim 41, wherein the material is bioabsorbable material.

46. The method according to claim 41, wherein the first end and the second end of the flat design are coupled by welding.

47. A method for constructing an apparatus for fixation of a sensor in a bodily lumen, comprising the steps of:
cutting a flat design of both a fixation device and a sensor support from a portion of material with a laser, the flat design of the fixation device having a first end and a second end; and
coupling the first end and the second end of the flat design of the fixation device.

48. A method for remotely monitoring at least one physiological parameter within a bodily lumen, comprising the steps of:
fixing at least one remotely monitorable sensor within the bodily lumen; and
interrogating the at least one sensor from outside the bodily lumen.

49. The method according to claim 48 wherein the at least one sensor is fixed within the bodily lumen using a fixation device having a sensor support.

50. The method according to claim 49, wherein the fixation device is a stent.

51. The method according to claim 49, wherein the fixation device is an anchoring ring.

52. The method according to claim 48, wherein the interrogating is periodic.

53. The method according to claim 48, wherein the interrogating is continuous.

54. The method according to claim 48, wherein the interrogating is done from the surface of the body.

55. A method for fixation of a sensor in a bodily lumen, comprising the steps of:
applying a soluble protective layer to the sensor;
placing the sensor into a sensor support coupled to a fixation device;
inserting the fixation device into a bodily lumen; and
securing the fixation device within the bodily lumen.

56. The method according to claim 55, wherein the protective layer is a hard coating.

57. The method according to claim 55, wherein the protective layer is comprised of glucose and sucrose.

58. The method according to claim 57, wherein the glucose and sucrose are of approximately equal proportions.

59. The method according to claim 55, wherein the protective layer is comprised of finely ground Hydroxy Propyl Methyl Cellulose, Hydroxy Propyl Cellulose and Colloidal Silicone Dioxide, and water.

60. The method according to claim 55, wherein the protective layer is soluble in an aqueous solution.

61. The method according to claim 55, wherein the protective layer dissolves rapidly after insertion.

62. The method according to claim 55, wherein the protective layer is applied by spraying the sensor.

63. The method according to claim 55, wherein the protective layer is applied by dipping the sensor.

64. The method according to claim 55, wherein the protective layer is applied by painting the sensor.

65. A protective layer for a sensor inserted within a bodily lumen comprising:
glucose; and
sucrose, wherein the glucose and sucrose are of approximately equal proportions.

66. A protective layer for a sensor inserted within a bodily lumen comprising:
finely ground hydroxy propyl methyl cellulose;
finely ground hydroxy propyl cellulose;
finely ground colloidal silicone dioxide; and
water.

67. A method for protecting a remotely monitorable sensor from damage during insertion into and fixation in a bodily lumen, comprising the step of applying a soluble protective layer to the sensor, wherein the protective layer dissolves after insertion and fixation to allow proper operation of the sensor.

68. The method according to claim 66, wherein the soluble protective layer further comprising glucose and sucrose, wherein the glucose and sucrose are of approximately equal proportions.

69. The method according to claim 66, wherein the soluble protective layer further comprising finely ground hydroxy propyl methyl cellulose, finely ground hydroxy propyl cellulose, finely ground colloidal silicone dioxide, and water.
